**Europäisches Patentamt**

**European Patent Office**

**Office européen des brevets**

(19)

(11) Numéro de publication : **0 329 784 B1**

(12) # FASCICULE DE BREVET EUROPEEN

(45) Date de publication du fascicule du brevet :
**05.02.92 Bulletin 92/06**

(51) Int. Cl.⁵ : **A61M 5/14**

(21) Numéro de dépôt : **89900610.0**

(22) Date de dépôt : **25.07.88**

(86) Numéro de dépôt international :
**PCT/FR88/00386**

(87) Numéro de publication internationale :
**WO 89/00866 09.02.89 Gazette 89/04**

(54) **DISPOSITIF ET PROCEDE POUR L'ADMINISTRATION DE MEDICAMENTS FLUIDES, NOTAMMENT POUR LA PERFUSION.**

(30) Priorité : **31.07.87 FR 8710881**

(43) Date de publication de la demande :
**30.08.89 Bulletin 89/35**

(45) Mention de la délivrance du brevet :
**05.02.92 Bulletin 92/06**

(84) Etats contractants désignés :
**AT BE CH DE FR GB IT LI LU NL SE**

(56) Documents cités :
**GB-A- 2 040 379**
**GB-A- 2 063 684**
**US-A- 4 201 207**
**US-A- 4 298 000**
**US-A- 4 346 703**

(73) Titulaire : **SOCIETE DE PRODUITS POUR L'INDUSTRIE LA RECHERCHE ET LES ANALYSES DE LABORATOIRES (S.P.I.R.A.L.)**
**3, rue des Mardors, Couternon**
**F-21560 Arc-sur-Tille (FR)**

(72) Inventeur : **PEREGO, Jean**
**Château Preville Ouges**
**F-21600 Longvic (FR)**
Inventeur : **PEREGO, François, Bernard**
**6, rue de la Libération**
**Béchérel F-35190 Tinténiac (FR)**
Inventeur : **GUYONDET, Patrick**
**8, rue du Varin**
**F-21560 Brosey-sur-Tille (FR)**

(74) Mandataire : **Bertrand, Didier et al**
**c/o S.A. Fedit-Loriot 38, avenue Hoche**
**F-75008 Paris (FR)**

EP 0 329 784 B1

## Description

## DOMAINE DE L'INVENTION

La présente invention a trait au domaine de l'administration d'une préparation médicamenteuse de préférence (i) en quantité relativement faible et (ii) pendant les durées de temps relativement longues.

Plus précisément elle concerne un procédé de distribution vers son site d'administration d'une préparation médicamenteuse liquide et un dispositif d'administration qui sont utilisables principalement dans le domaine de la perfusion artérielle, veineuse ou sous-cutanée, et, également dans celui de l'administration de principes actifs par voie percutanée où lesdits principes actifs disposés au contact de la peau traversent la peau pour pénétrer dans l'organisme.

Grâce à ce procédé et à ce dispositif l'on administre au patient, notamment par perfusion, le strict nécessaire de préparation médicamenteuse.

## ART ANTERIEUR

On sait que, en milieu hospitalier, on a utilisé jusqu'à ce jour deux appareillages pour la perfusion, à savoir: le goutte à goutte et le pousse-seringue.

Le goutte à goutte, qui est souvent utilisé car très économique, a un débit très aléatoire, le volume de liquide de perfusion administré pouvant varier de ± 150% dès lors que le réglage est manuel. Le goutte à goutte présente plusieurs autres inconvénients, à savoir notamment (i) l'introduction dans la circulation générale du patient d'un volume important de liquide (en général isotonique) pas toujours souhaitable, en particulier lorsque le patient est atteint de troubles rénaux ou autres empêchant ou limitant la miction, et (ii) l'immobilisation du patient.

En revanche, le pousse-seringue est théoriquement très efficace en ce qui concerne la précision du débit quand il n'est pas en panne ; il fournit un débit régulier et n'introduit dans la circulation générale que la stricte quantité requise de liquide de perfusion. Malheureusement, le pousse-seringue présente de nombreux inconvénients : il implique l'immobilisation du patient, est relativement très onéreux (environ 6.000, -- FF l'unité), tombe souvent en panne et exige un personnel technique hautement qualifié pour les réglages (pas toujours faciles) des débits requis.

On sait également que l'on a proposé, en ce qui concerne la technique du goutte à goutte, des dispositifs pour la perfusion ambulatoire continue des patients. On connaît notamment de FR-A-2447198, FR-A-2447199 et FR-A-2499857 un dispositif comprenant un récipient pliable et souple de solution à administrer, un orifice de transfert lié audit récipient, et, un tube souple allant dudit orifice au cathéter du patient et qui est pourvu d'un élément frangible.

On connaît de US-A-4298000 un dispositif de perfusion, dans lequel un capillaire pour régulariser le débit de la préparation médicamenteuse à administrer est disposé en aval du réservoir contenant ladite préparation.

Ce document ne décrit ni ne suggère l'utilisation, en amont dudit réservoir, (i) d'un accumulateur hydraulique du type comprenant une membrane souple montée de façon étanche autour dudit dispositif, et (ii) d'un capillaire régulant l'admission du fluide hydraulique pour pousser la préparation à perfuser vers son site d'administration.

On connaît également de US-A-4201207 un autre dispositif de perfusion dans lequel la préparation à administrer est logée dans une enveloppe en caoutchouc (ou "vessie", en anglais "bladder") qui est comprimée et qui se déverse dans un organe de distribution par l'intermédiaire d'une aiguille capillaire. Ce document ne décrit ni ne suggère l'utilisation d'un accumulateur hydraulique agissant en amont du réservoir contenant la préparation à perfuser par l'intermédiaire d'un capillaire régulant l'admission du fluide hydraulique sollicitant un piston poussant ladite préparation vers son site d'administration.

On connaît enfin du quotidien "Le Monde" daté du mercredi 22 avril 1987, page 23 (publié le 21 avril 1987) qu'un perfuseur pour le traitement chimiothérapeutique a été préconisé. Ledit perfuseur, dont le principe repose sur la rétractation d'une membrane en latex, comprend une grosse seringue munie d'un réservoir en forme de ballon d'une capacité de 70 ml, et, un contrôleur de débit pourvu, semble-t-il, d'une membrane filtrante de 5 µm d'ouverture de maille. Par l'intermédiaire de ladite membrane filtrante, ledit perfuseur délivre 2 ml/h de solution médicamenteuse à administrer au moyen d'un cathéter par voie veineuse, artérielle ou sous-cutanée. Or, la seule rétraction d'une membrane en latex n'assure pas la régularité du débit de la préparation médicamenteuse à distribuer.

## BUT DE L'INVENTION

Il existe dans le domaine de la perfusion un besoin en appareillage simple d'emploi, efficace et peu onéreux, permettant de limiter la quantité de liquide à administrer au strict minimum.

Selon l'invention on propose une nouvelle solution technique qui convient pour la perfusion artérielle, veineuse ou sous-cutanée ainsi que pour l'administration percutanée également appelée transcutanée. Cette nouvelle solution technique présente les avantages du pousse-seringue, en ce qui concerne la régularité du débit et le strict volume de préparation médicamenteuse à administrer, et, du goutte à goutte, en ce qui concerne l'aspect économique, sans les inconvénients susmentionnés desdits pousse-seringue et goutte à goutte.

Cette nouvelle solution technique s'appuie, comme le perfuseur décrit dans "Le Monde", sur la rétractation d'une membrane élastique, elle s'en distingue cependant par la mise en oeuvre de moyens différents, notamment la présence d'un conduit capillaire dans le système hydraulique freinant la circulation du fluide hydraulique. Le débit de la préparation médicamenteuse à administrer selon l'invention est régularisé au niveau du système hydraulique par le capillaire et la viscosité du fluide hydraulique intervenant comme fluide moteur ou propulseur.

Cette nouvelle solution technique offre l'intérêt de pouvoir être appliquée aussi bien au patient hospitalisé qu'au patient ambulatoire. Elle permet en particulier de réaliser des perfusions de façon continue à des débits très faibles, par exemple inférieurs à 1 ml par 24 h, en particulier des débits de l'ordre de 1 ml/72 h et même d'obtenir, dans le cas d'un dispositif implanté dans l'organisme, des débits de l'ordre de 10 ml /an voire encore de 5 à 1 ml/an si nécessaire. Des débits aussi faibles diminuent fortement la toxicité des principes actifs souvent administrés dans le passé par injections massives et successives.

## RESUME DE L'INVENTION

Suivant l'invention on préconise donc un procédé de distribution vers son site d'administration d'une préparation médicamenteuse liquide, l'administration pouvant notamment se faire par perfusion, par implantation ou par voie percutanée, sous un débit déterminé pendant une durée de temps relativement importante, ledit procédé de distribution, dans lequel on utilise la rétractation d'une membrane élastique, étant caractérisé en ce que l'on place ladite préparation médicamenteuse dans un réservoir tubulaire où elle est soumise à l'action d'au moins une paroi mobile sollicitée par la pression d'un fluide hydraulique dont on contrôle l'admission par un conduit capillaire alimenté en fluide hydraulique sous pression par un accumulateur hydraulique sollicité par une membrane élastique rétractable préalablement dilatée, ledit réservoir étant logé dans un évidement longitudinal débouchant à l'extérieur et creusé dans un corps cylindrique, ladite membrane élastique étant montée de façon étanche sur au moins une portion de la surface extérieure dudit corps cylindrique, ledit accumulateur hydraulique comprenant un fluide hydraulique logeable dans l'espace situé entre (a) ladite membrane élastique dilatée par ledit fluide et (b) ladite portion de surface extérieure dudit corps cylindrique recouverte par ladite membrane élastique.

Suivant l'invention, on préconise également, en tant que produit industriel nouveau un dispositif pour l'administration en continu d'une préparation médicamenteuse liquide, notamment par perfusion, par implantation ou par voie percutanée, ledit dispositif, qui comporte un système hydraulique et un réservoir et dans lequel le fluide hydraulique mis en circulation par la rétractation d'une membrane élastique préalablement dilatée assure, par l'intermédiaire d'un piston, la distribution en continu de ladite préparation contenue dans ledit réservoir vers son site d'administration, étant caractérisé en ce qu'il comprend dans ledit système hydraulique un accumulateur hydraulique sollicité par une membrane élastique rétractable, un conduit capillaire alimenté en fluide hydraulique par ledit accumulateur hydraulique, et un piston disposé en aval dudit conduit capillaire et sollicité par le fluide hydraulique provenant dudit conduit capillaire, ledit piston étant destiné au transfert en continu de la préparation médicamenteuse contenue dans ledit réservoir vers ledit site d'administration, ledit réservoir étant logé dans un évidement longitudinal débouchant à l'extérieur et creusé dans un corps cylindrique, ladite membrane élastique étant montée de façon étanche sur au moins une portion de la surface extérieure dudit corps cylindrique, ledit accumulateur hydraulique comprenant un fluide hydraulique logeable dans l'espace situé entre (a) ladite membrane élastique dilatée par ledit fluide et (b) ladite portion de surface extérieure dudit corps cylindrique recouverte par ladite membrane élastique.

## DESCRIPTION DETAILLEE DE L'INVENTION

Le débit de la préparation médicamenteuse à distribuer à son site d'administration est régularisé par le diamètre du conduit capillaire et la viscosité du fluide hydraulique. De façon pratique, le diamètre interne du conduit capillaire se situera, pour un fluide hydraulique tel que l'eau et les mélanges eau-éthylèneglycol et eau-propylèneglycol, dans la plage allant de 10 μm à 90 μm et de préférence entre 30 μm et 80 μm, et pour un fluide hydraulique de viscosité plus élevée tel que l'huile de silicone et l'huile de jojoba, dans la plage allant de 100 à 900 μm. Il est important, ainsi qu'on le verra plus loin, que ledit diamètre soit essentiellement constant sur toute la longueur du conduit de son entrée à sa sortie. En vue de standardiser la production industrielle, on utilisera avantageusement des conduits capillaires ayant des diamètres et des hauteurs rigoureusement fixés pour l'administration d'un volume donné de préparation médicamenteuse pendant une durée donnée.

Le fluide hydraulique sera de façon pratique un liquide essentiellement non compressible ou peu compressible. Convient notamment à cet effet un liquide choisi parmi l'ensemble comprenant l'eau, les solutions aqueuses salines, les huiles (par exemple l'huile de paraffine, de palme, d'arachide, de lin, d'olive, de jojoba etc.), les alkylèneglycols tels que les éthylèneglycol et propylèneglycol, les polyalkylèneglycols (tels que les polyéthylèneglycols et polypropylèneglycols liquides dans les conditions usuelles de température et de pression), les silicones et leurs mélanges. Le cas échéant, le fluide hydraulique pourra être gazeux. Cependant on préfère les fluides liquides aux fluides gazeux. D'une manière générale, suivant l'invention le fluide hydraulique ne sera jamais en contact avec la préparation médicamenteuse à administer.

Suivant le meilleur mode de mise en oeuvre de l'invention, on préconise un dispositif qui comprend :
— un corps cylindrique creusé d'un évidement longitudinal débouchant à l'extérieur et dont le fond présente une canalisation d'amenée de fluide hydraulique,
— une membrane élastique montée de façon étanche sur au moins une portion de la surface extérieure dudit corps cylindrique,
— un accumulateur hydraulique comprenant un fluide hydraulique logeable dans l'espace situé entre (a) ladite membrane élastique dilatée par ledit fluide et (b) ladite portion de surface extérieure dudit corps cylindrique recouverte par ladite membrane élastique,
— un conduit capillaire destiné à être alimenté en fluide hydraulique par ledit accumulateur hydraulique, d'une part, et à alimenter ladite canalisation d'amenée disposée en aval dudit capillaire au fond de l'évidement dudit corps cylindrique, d'autre part,
— au moins un piston situé dans ledit évidement du corps cylindrique et susceptible de se déplacer axialement à l'intérieur dudit évidement, ledit piston comprenant une première face distale directement sollicitée par le fluide hydraulique provenant de ladite canalisation d'amenée de fluide et une seconde face distale intervenant pour la distribution en continu de ladite préparation médicamenteuse vers son site d'administration,
— un réservoir tubulaire contenant la préparation médicamenteuse liquide à administrer,
ledit réservoir et ledit système hydraulique étant agencés de telle façon que ledit réservoir tubulaire soit solidaire du corps cylindrique et associé audit piston.

D'autre avantages et caractéristiques de l'invention seront mieux compris à la lecture qui va suivre d'exemples de réalisation, nullement limitatifs mais donnés à titre d'illustration, en liaison avec les dessins annexés dans lesquels :
— la figure 1 est une vue en coupe d'un premier dispositif conforme à l'invention ;
— la figure 2 est une vue en coupe du corps cylindrique du dispositif de la figure 1 ;
— la figure 3 est une vue en coupe schématique d'un second dispositif conforme à l'invention et différent de celui de la figure 1 notamment par la configuration de la membrane rétractable ;
— les figures 4 et 5 illustrent schématiquement les modalités de manipulation préalable des réservoirs des dispositifs des figures 1 et 3 avant la mise en service desdits dispositifs ;
— la figure 6 illustre schématiquement l'utilisation d'un dispositif analogue à celui de la figure 1 agencé en pousse-seringue ;
— la figure 7 illustre un mode d'administration percutané au moyen d'un dispositif suivant l'invention ; et,
— la figure 8 illustre un mode de réalisation du conduit capillaire de l'invention.

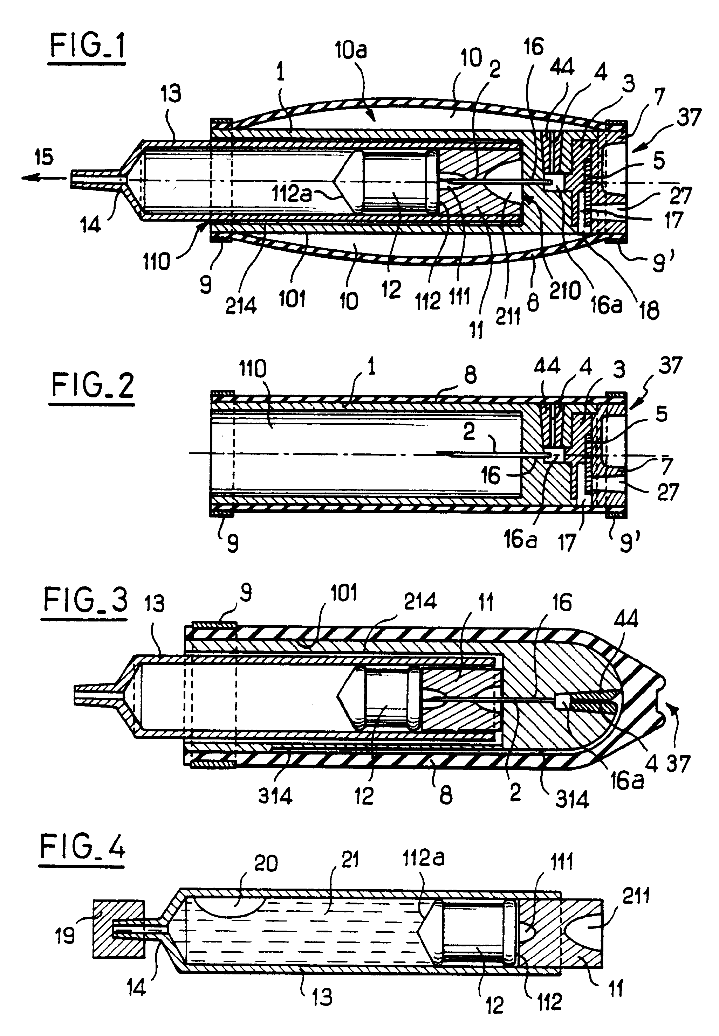

Le dispositif conforme à l'invention comprend (a) un réservoir tubulaire 13 contenant la préparation médicamenteuse à administrer (désignée 21 dans la figure 4) et (b) un système hydraulique qui comporte principalement un accumulateur hydraulique 10 lors de la mise en service pour le transfert de la préparation médicamenteuse vers son site d'administration, une membrane élastique 8 agissent en continu, lors de sa rétractation, sur le fluide hydraulique 10 afin d'assurer sa circulation dans ledit système hydraulique, un accumulateur hydraulique sollicité par ladite membrane 8, un conduit capillaire 4 alimenté en fluide hydraulique par ledit accumulateur, un piston 12 sollicité par ledit fluide hydraulique provenant dudit conduit capillaire 4 et au moins un moyen, notamment la conduite d'arrivée 16, pour conduire à l'intérieur du corps cylindrique 1 ledit fluide hydraulique de la sortie du capillaire 4 vers la face distale 112 dudit piston 12.

Le corps cylindrique 1 assure une fonction statique, la fixation du réservoir 13, et une fonction dynamique, la coopération du système hydraulique et dudit réservoir pour le transfert en continu de la préparation médicamenteuse vers son site d'administration où ladite préparation est introduite dans le corps humain en continu.

De façon pratique, il est agencé de façon à permettre la circulation du fluide hydraulique de l'accumulateur hydraulique vers le piston 12.

Ledit corps cylindrique 1 comprend (voir figures 1, 2, 3 et 6) un premier évidement longitudinal 110 débouchant à l'extérieur et dont l'axe est parallèle ou identique à celui dudit corps cylindrique. Le fond de l'évidement 110 comporte une canalisation d'amenée 16 de fluide hydraulique de façon que celui-ci agisse sur la face distale 112 du piston 12.

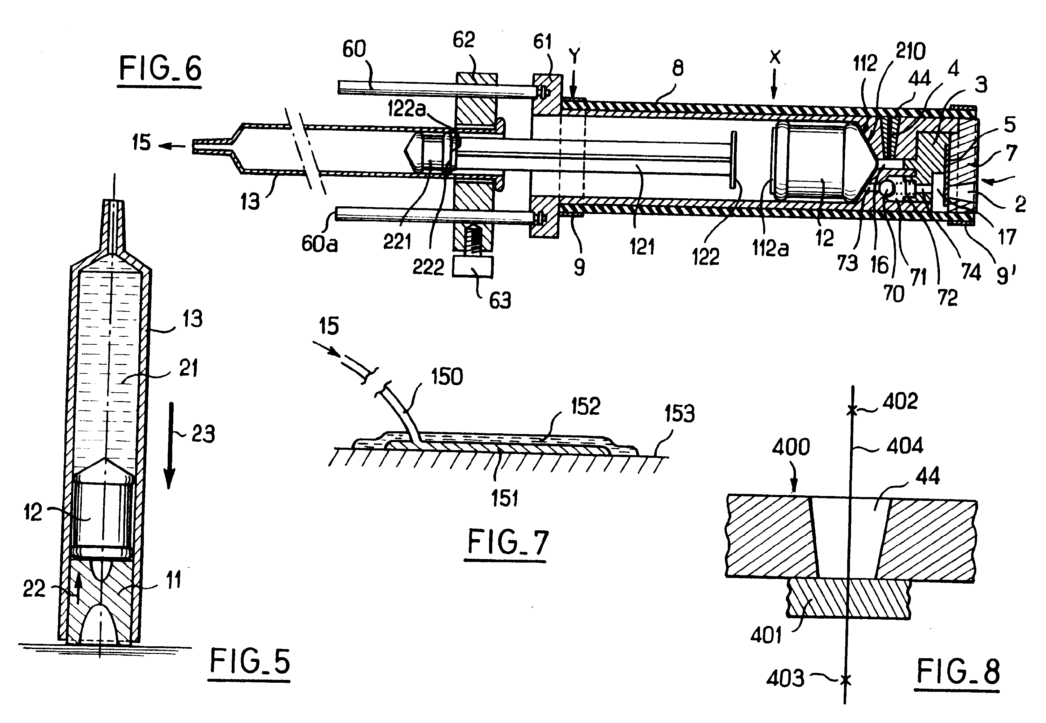

Du côté opposé au premier évidement 110, le corps cylindrique 1 comporte plusieurs autres évidements: un évidement pour le logement du dispositif capillaire 44 comprenant le conduit capillaire 4 et au moins un évidement pour loger un système de chargement 37 de l'accumulateur hydraulique en fluide hydraulique, et le cas échéant, au moins un évidement pour le recyclage du fluide hydraulique (voir figure 6), après administration du contenu du réservoir 13, afin de faire revenir ledit fluide dans l'accumulateur hydraulique.

Comme représenté par les figures 1, 2 et 6, le système de chargement ou d'introduction 37 du fluide hydraulique dans le système hydraulique peut être du type comprenant une pièce 7 pouvant être logée dans le corps 1 au voisinage de l'extrémité de l'évidement opposé à l'évidement 110. Cette pièce 7 est pourvue par exemple d'un canal d'introduction 27, notamment par injection au moyen d'une seringue ou de tout autre dispositif approprié. Le canal 27 est obturable intérieurement par un clapet anti-retour 5 ou un moyen analogue. Une cloison 3 est disposée dans le corps 1 derrière ladite pièce 7. Cette cloison réalise une séparation entre (i) le circuit de chargement du fluide hydraulique qui comprend la cavité interne 17 située dans l'espace compris entre la pièce 7, le clapet 5 et la cloison 3, d'une part, et la fenêtre 18 prévue dans le corps 1 pour l'alimentation de l'espace 10a de l'accumulateur hydraulique, d'autre part, et (ii) la portion de circuit du système hydraulique comprenant soit la canalisation d'amenée 16 seule (figure 6) soit l'ensemble de ladite canalisation 16 et la cavité 16a disposée entre la sortie du conduit capillaire 4 et ladite canalisation 16 (figures 1, 2 et 3).

L'introduction du fluide hydraulique par l'intermédiaire du système 37 des figures 1, 2 et 6 est avantageusement réalisée au moyen d'une seringue dépourvue d'aiguille et contenant ledit fluide. On introduit l'embout de la seringue dans le canal 27. Cette opération commande l'ouverture du clapet 5 généralement conçu en caoutchouc ou autres matériaux appropriés. En poussant le mandrin de cette seringue on fait passer ledit fluide qu'elle contient dans la cavité 17 puis, par l'intermédiaire de la fenêtre 18 prévue au voisinage de la surface 101 du corps cylindrique 1, dans l'espace 10a. Au fur et à mesure que l'espace 10a se remplit, la membrane 8 se dilate. Si on veut arrêter l'administration de la préparation médicamenteuse 21 contenue dans le réservoir tubulaire 13 avant la fin de ladite administration, on introduit l'embout de la seringue vide dépourvue d'aiguille dans le canal 27, et, par la pression de l'accumulateur hydraulique tout le fluide 10 contenu dans l'espace 10a passe dans ladite seringue.

Suivant une variante représentée à la figure 3 de ce système de chargement de fluide hydraulique, le site d'introduction 37 est constitué par l'extrémité arrondie du capuchon formant la membrane 8. Dans ce cas d'espèce, l'introduction du fluide hydraulique 37 est réalisée au moyen d'une seringue pourvue d'une aiguille traversant ladite membrane au niveau du site 37.

Selon un mode de réalisation représenté par les figures 1, 2 et 3, la canalisation d'amenée 16 de fluide hydraulique du corps cylindrique 1 comporte sur au moins une tartie de sa longueur une aiguille creuse 2 se prolongeant du côté de sa pointe effilée à l'intérieur de l'évidement 110 dudit corps cylindrique, ladite aiguille creuse 2 étant associée de façon étanche à ladite canalisation 16 et intervenant pour l'alimentation en fluide hydraulique de la paroi 112 du piston 2, d'une part, et pour la fixation du réservoir tubulaire 13 solidairement audit corps cylindrique 1, d'autre part.

Suivant ce mode de réalisation, la fixation du réservoir tubulaire 13 au corps cylindrique 1 au moyen de l'aiguille creuse 2 est réalisée grâce à un bouchon 11 obturant ledit réservoir 13 du côté opposé à celui de l'administration, ledit bouchon qui est traversé de façon étanche par ladite aiguille, étant logé au moins sur une partie de son épaisseur dans l'intérieur dudit réservoir 13 et disposé en amont du piston 12 lequel est susceptible de coulisser de façon étanche à l'intérieur dudit réservoir 13, la pointe effilée de ladite aiguille 2 émergeant dudit bouchon au voisinage de la paroi 112 dudit piston.

De façon pratique le bouchon 11 est pourvu de deux évidements coaxiaux opposés 111 et 211 destinés à diminuer l'épaisseur de la matière devant être traversée par la pointe effilée de l'aiguille 2.

L'accumulateur hydraulique comporte, comme indiqué ci-dessus, une membrane élastiquement rétractable 8 sertie ou montée de façon étanche sur au moins une partie de la surface externe 101 du corps 1 par l'intermédiaire d'au moins une bague 9. En pratique, la membrane élastiquement rétractable 8 est prévue pour s'appliquer sur la plus grande partie de ladite surface 101 avant l'introduction du fluide hydraulique dans l'espace 10a et après administration du contenu du réservoir tubulaire 13. La membrane 8 peut, comme illustré par les figures 1, 2 et 6, constituer un manchon assujetti de façon étanche à la paroi 101 par deux bagues 9 et 9′ disposées avantageusement chacune au voisinage d'une extrémité du corps cylindrique 1, ou, comme représenté à la figure 3 un capuchon enrobant également le site 37 d'introduction du fluide hydraulique dans le système, ledit capuchon étant assujetti de façon étanche par une seule bague 9 à la surface 101 au niveau de son ouverture vers l'extérieur au voisinage de l'ouverture de l'évidement 110 du corps 1.

De façon pratique, on préfère que le diamètre interne du manchon selon les figures 1, 2 et 6, d'une part, et du capuchon selon la figure 3, d'autre part, soit à l'état normal inférieur au diamètre extérieur du corps cylindrique 1. Un tel agencement assure une pression sensiblement uniforme sur la surface 101 du corps 1 jusqu'à ce que pratiquement tout le fluide 10 contenu dans l'espace 10a ait été utilisé pour l'administration du volume total de la préparation médicamenteuse 21.

Suivant l'invention pour assurer un bon écoulement du fluide 10 vers le conduit capillaire 4 en fin d'administration, quand la membrane 8 vient au contact de la surface 101, on préconise l'utilisation d'une surface 101 comprenant une ou plusieurs rigoles 314 (voir figure 3), par exemple une ou plusieurs rigoles longitudinales ou encore une rigole disposée en spirale le long de la surface 101 et intervenant en tant que collecteur de fluide vers l'entrée du conduit capillaire 4.

La membrane 8 qui est rétractable après avoir été dilatée par l'introduction de fluide hydraulique 10 dans l'espace 10a est choisie parmi les matériaux présentant un module d'élasticité élevé (grande élongation avant rupture) et une aptitude de retour aux dimensions d'origine après élongation prolongée. Conviennent à cet effet le caoutchouc naturel ou synthétique obtenu notamment par vulcanisation de latex naturel, de néoprène, de polybutadiène, ou encore le caoutchouc silicone. Le caoutchouc obtenu à partir de latex naturel et concentré par l'évaporation est auto-vulcanisable notamment celui connu sous la dénomination commerciale de REVULTEX® MR. Ce matériau présente l'avantage d'être relativement stable au stockage tant à vide que sous pression en ce sens qu'il résiste au vieillissement par oxydation (il comporte en effet des agents antioxydants). Comme son rapport pression à vide/pression en charge est le plus proche de la valeur 1, le débit de l'administration de la préparation 21, notamment par perfusion, sera sensiblement identique du début à la fin. Par exemple, avec le matériau REVULTEX® MR précité, pour un dispositif d'administration de 10 ml de préparation médicamenteuse 21 la pression à vide est approximativement de 0,6 kg/cm$^2$ et la pression en charge est approximativement de 0,65 kg/cm$^2$, soit un écart de 8,3% ; pour un dispositif d'administration de 5 ml de préparation médicamenteuse 21, la pression à vide est approximativement de 0,75 kg/cm$^2$ et la pression en charge est approximativement de 0,8 kg/cm$^2$ soit un écart de 6,6%. En choisissant l'épaisseur du manchon ou du capuchon, d'une part, et l'élongation permanente dudit manchon ou dudit capuchon, d'autre part, on obtient la pression à vide désirée. Ainsi avec le dispositif de la figure 1, si l'épaisseur du manchon en caoutchouc REVULTEX® MR précité est de 1,4 mm, le diamètre du manchon avant montage est de 13,3 mm et le diamètre extérieur du corps 1 est de 20 mm, l'élongation permanente du manchon est de 150%.

Dans le dispositif de l'invention, la membrane 8 se présente donc sous la forme d'un manchon ou capuchon disposé autour du corps cylindrique 1. Suivant une des caractéristiques de l'invention, le diamètre dudit manchon ou capuchon à l'état non étiré est inférieur à celui de la surface 101 dudit corps 1, le rapport :

$$R = \frac{\text{pression en charge} - \text{pression à vide}}{\text{pression à vide}}$$

de ladite membrane 8 disposée autour dudit corps 1 est compris entre 0,05 et 0.10 et avantageusement entre 0,06 et 0,09.

Ainsi lorsque l'espace 10a est dépourvu de fluide 10, la pression à vide de la membrane 8 n'est pas nulle.

Dans l'espace 10a le fluide hydraulique 10 est confiné par la membrane 8 la surface 1 et le conduit capillaire 4 freinant l'écoulement dudit fluide sous la pression de ladite membrane 8 vers la canalisation 16 et la paroi 112 du piston 12.

Suivant l'invention le conduit capillaire 4 a un diamètre régulier et est réalisé dans une pièce 44 logeable de façon étanche dans un évidement approprié du corps cylindrique 1 entre l'accumulateur hydraulique et la canalisation d'amenée 16.

Le débit étant régi par la pression du fluide dans l'espace 10a, la viscosité du fluide hydraulique et le diamètre du capillaire, il est important que le diamètre du capillaire soit réalisé avec la plus grande précision possible. La précision requise est illustrée par le tableau I ci-après donnant les variations de sections de capillaires en fonction de variations de diamètres de 1 μm.

EP 0 329 784 B1

## TABLEAU I

| Variation du diamètre capillaire | Diamètre (μm) | Sections (μm²) | Variation des sections (%) |
|---|---|---|---|
| 10μm±1μm | 11 | 950 | + 21 |
| | 10 | 785 | 0 |
| | 9 | 636 | - 19 |
| 40μm±1μm | 41 | 1 320 | + 5 |
| | 40 | 1 257 | 0 |
| | 39 | 1 194 | - 5 |
| 80μm±1μm | 81 | 5 152 | + 2,6 |
| | 80 | 5 020 | 0 |
| | 79 | 4 901 | - 2,4 |

Le diamètre du conduit capillaire 4 suivant l'invention a une tolérance inférieure à 1 μm. Selon un mode préféré de réalisation on part d'une pièce 44 en quartz dans laquelle on perce le conduit 4 suivant le diamètre requis avec une précision inférieure à 1 μm, par exemple en utilisant une pièce de quartz monocristalline. Selon une autre variante de réalisation également préféré selon l'invention, on dispose, comme représenté à la figure 8 un fil métallique 404 étiré ayant un diamètre déterminé à ± 1 μm que l'on tend entre des organes représentés par 402 et 403. Le fil 404 est disposé suivant l'axe d'un moule 400 de forme tronconique dont la section inférieure qui est la plus petite est obturée par une pièce 401 appropriée. On coule dans la cuvette 44 de ce moule un volume constant d'un alliage à bas point de fusion par exemple l'alliage de WOOD (qui fond à 70°C), l'alliage de DARCET (qui fond à 100°C) ou encore un matériau plastique notamment réticulable au moyen d'une substance ou catalyseur appropriés. Après solidification il suffit de retirer le fil 404 qui est généralement conçu en acier. Le conduit capillaire de haute précision suivant l'invention est démoulé et prêt à être monté.

De façon pratique, la cuvette 44 peut présenter une hauteur comprise entre 0,5 et 20 mm et, le cas échéant, une conicité de l'ordre de 2% à 10%, le diamètre inférieur de ladite cuvette pouvant être de l'ordre de 2 mm. Comme indiqué ci-dessus le diamètre intérieur du conduit capillaire 4 est compris entre 10 et 900 μm.

Le réservoir tubulaire 13, qui a une forme cylindrique, présente à l'une de ses extrémités un rétrécissement du type cône de LUER et est solidaire du corps cylindrique 1, son contenu 21 étant sollicité soit directement soit indirectement par la cloison 112a du piston 12 par son autre extrémité.

Suivant un mode de réalisation conforme à l'invention, l'intérieur du réservoir tubulaire 13 peut contenir le piston 12. En d'autres termes, ledit piston 12 se trouvant à l'intérieur de l'évidement 110 du corps cylindrique 1 est agencé de façon à être susceptible de coulisser à l'intérieur du réservoir 13 contenant la préparation médicamenteuse 21.

Comme indiqué ci-dessus, l'aiguille creuse 2 intervient dans ce mode de réalisation comme support du réservoir tubulaire 13 par l'intermédiaire du bouchon 11 et comme moyen d'amenée de fluide hydraulique sur la face 112 du piston 12.

Selon un autre mode de réalisation, le réservoir tubulaire 13 est solidaire du corps 1 par l'intermédiaire

7

d'un système de fixation comprenant une collerette 61 prévue à l'extrémité du corps 1 au niveau de l'ouverture de l'évidement 110, au moins une tige 60, 60a associée à ladite collerette 61 et un moyen 62 du type étau bloquant la tige 60 et/ou la tige 60a par l'intermédiaire d'un organe approprié, par exemple une vis 63. Suivant ce mode, le piston 12 se trouvant à l'intérieur de l'évidement 110 du corps cylindrique 1 est agencé de façon à solliciter par la pression du fluide hydraulique sur sa paroi 112 un système de poussée 121, 221 agissant sur le contenu du réservoir 13, ledit système de poussée étant solidaire dudit réservoir 13, susceptible de coulisser à l'intérieur dudit réservoir 13 et logé au moins partiellement à l'intérieur dudit évidement 110.

Les figures 4 et 5 illustrent les modalités à suivre pour fixer le réservoir tubulaire 13 au moyen de l'aiguille 2. Lors du stockage de la préparation médicamenteuse 21 dans le réservoir 13 celui-ci est obturé par un bouchon 19 disposé sur le cône de LUER à l'une de ses extrémités, d'une part, et par un bouchon 11 en contact avec la face 112 du piston 12 logé dans ledit réservoir, d'autre part. Ledit bouchon 11 est partiellement logé dans le tube 13, une partie dudit bouchon s'étendant à l'extérieur du tube 13. La préparation 21 contenue dans ledit réservoir présente un ménisque ou bulle 20 (voir figure 4).

Avant la mise en service, le réservoir tubulaire 13 prérempli par la préparation 21 est manipulé, maintenu vertical, de façon à enlever le bouchon 19 du cône de LUER. On enfonce le bouchon 11 en exerçant une force suivant la flèche 23, la pénétration dudit bouchon 11 à l'intérieur du réservoir 13 chassant la bulle 20 par le cône de LUER et faisant monter le piston 12 suivant la flèche 22 (voir figure 5). On met alors en place le réservoir 13 dans l'évidement 110 de façon que l'aiguille 2 perce le bouchon 11 et maintienne en place ledit réservoir dans ledit corps.

Cette façon d'opérer permet de pallier l'inconvénient, qui peut se rencontrer assez souvent, de l'adhérence du piston 12 au réservoir tubulaire 13 lors du stockage. La poussée suivant la flèche 23 permet la suppression de ladite adhérence. Suivant ce mode opératoire, lorsque le réservoir 13 est maintenu par le bouchon 19 perforé par l'aiguille creuse 2, la paroi externe dudit réservoir peut se trouver à une certaine distance de la paroi 214 de l'évidement 110.

Le dispositif représenté par la figure 6 est agencé en pousse-seringue. Plus précisément le réservoir 13 est monté dans une seringue, la préparation médicamenteuse 21 contenue dans ledit réservoir étant sollicitée par la pression du fluide hydraulique exercée sur la paroi amont 112 (qui se déplace de la position X vers la position Y pour la distribution de la préparation 21) du piston 12 et transmise par l'intermédiaire d'un mandrin 121 mû par la paroi amont 112a dudit piston 12. Ici, la paroi 112a du piston 12 agit sur la platine amont 122 du mandrin 121 et la platine 122a dudit mandrin agit sur la face amont 222 du piston 221 logé dans le réservoir tubulaire 13.

Dans cette variante, ledit mandrin 121 sollicite un piston 221 susceptible de coulisser à l'intérieur du réservoir 13, ledit réservoir étant solidaire dudit corps cylindrique 1 par l'intermédiaire d'un système de fixation représenté schématiquement par les organes 60, 62, 63 lié à l'ouverture extérieure de l'évidement 110 par la collerette 61 dudit corps cylindrique 1.

Selon une autre caractéristique de l'invention, le dispositif d'administration est tel que le fluide hydraulique 10 soit recyclable. Le dispositif représenté à la figure 6 comporte un système de recyclage du type comprenant une bille 70 assujettie à un ressort 72 approprié tous deux logés dans un évidement 71 du corps cylindrique 1. Lors de l'administration, la bille 70 soumise à l'action du ressort 72 obture la canalisation 73 communiquant avec le fond 210 de l'évidement 110. Lors du recyclage en repoussant le piston 12 par un moyen approprié notamment une tige non représentée ici la bille 70, sollicitée par une pression supérieure à celle du ressort 72, libère la sortie de la canalisation 73 pour permettre le passage du fluide hydraulique 10 dans la cavité 17 par l'intermédiaire de la conduite 74 puis dans l'espace 10a par l'intermédiaire de la fenêtre 18.

Selon un autre mode de réalisation préféré de l'invention, la face 112 du piston 12 est rainurée ou striée de façon à mieux assurer l'entrainement du piston 12 par le fluide 10.

Comme illustré par la figure 6, la paroi 112 du piston 12 peut présenter une surface homologue à celle du fond 210 de l'évidement 110.

Le dispositif selon l'invention peut être utilisé pour administration d'une préparation médicamenteuse par perfusion, soit par voie intraveineuse, soit par voie intraartérielle, soit par voie intramusculaire, soit encore par voie intradermique ou sous-cutanée. On a obtenu de bons résultats avec le dispositif de la figure 1 par perfusion intradermique. Pour la perfusion, le cône de LUER 14 est relié par un tube souple au cathéter du patient.

Le dispositif de la figure 1 peut également être utilisé pour administration percutanée comme représenté par la figure 7, où un tube de faible diamètre 150 part du cône de LUER pour arriver à un matériau poreux 151 disposé au-dessus de la peau 153 et lié à celle-ci par l'intermédiaire d'un moyen de fixation 152 approprié notamment du type comprenant au moins une masse adhésive et au moins une pellicule protectrice qui peut être microperforée. Le matériau poreux 151 qui se présente sous la forme d'une plaque ou d'un disque de grande dimension par rapport à son épaisseur permet le passage du ou des principes actifs à travers la peau suivant un mécanisme bien connu de l'homme du métier (grande surface et faible épaisseur de matériau poreux imbibant la zone de la peau concernée par l'administration).

Le dispositif suivant l'invention, notamment celui représenté par la figure 1 et surtout par la figure 3, peut être utilisé pour administration au niveau de l'abdomen (par implantation intraabdominale en particulier), du rectum (par perfusion notamment), du scrotum (par perfusion ou par voie percutanée notamment).

De façon pratique, le volume total de fluide hydraulique 10 à introduire dans le dispositif de l'invention est sensiblement égal ou légèrement supérieur au volume de préparation médicamenteuse 21 à administrer suivant les réalisations représentées par les figures 1 et 3.

Dans le cas de la réalisation de la figure 6, on peut prévoir que le volume total de fluide hydraulique soit le cas échéant nettement supérieur au volume de la préparation 21 à administrer.

Quand il est utilisé pour administration par perfusion, le dispositif suivant l'invention peut, en raison de ses faibles dimensions, être porté dans une poche de vêtement, en pendentif autour du cour ou encore placé au voisinage du site d'introduction dans le cas d'une administration transrectale ou transscrotale.

Le dispositif suivant l'invention peut être fourni selon diverses tailles pour administration unique (dispositif jetable après utilisation) ou pour administrations multiples (dispositif avec recyclage du fluide hydraulique).

## Revendications

1. Procédé de distribution vers son site d'administration d'une préparation médicamenteuse liquide, l'administration pouvant notamment se faire par perfusion, par implantation ou par voie percutanée, sous un débit déterminé pendant une durée de temps relativement importante, ledit procédé de distribution, dans lequel on utilise la rétractation d'une membrane élastique, étant caractérisé en ce que l'on place ladite préparation médicamenteuse dans un réservoir tubulaire (13) où elle est soumise à l'action d'au moins une paroi mobile (112, 112a) sollicitée par la pression d'un fluide hydraulique (10) dont on contrôle l'admission par un conduit capillaire (4) alimenté en fluide hydraulique (10) sous pression par un accumulateur hydraulique sollicité par une membrane élastique (8) rétractable préalablement dilatée, ledit réservoir (13) étant logé dans un évidement longitudinal (110) débouchant à l'extérieur et creusé dans un corps cylindrique (1), ladite membrane élastique (8) étant montée de façon étanche sur au moins une portion de la surface extérieure (101) dudit corps cylindrique (1), ledit accumulateur hydraulique comprenant un fluide hydraulique (10) logeable dans l'espace (10a) situé entre (a) ladite membrane élastique dilatée par ledit fluide et (b) ladite portion de surface extérieure (101) dudit corps cylindrique (1) recouverte par ladite membrane élastique.

2. Procédé suivant la revendication 1, caractérisé en ce que le débit de la préparation médicamenteuse à distribuer à son site d'administration est régularisé par le diamètre du capillaire (4) et la viscosité du fluide hydraulique.

3. Procédé suivant l'une quelconque des revendications 1 et 2, caractérisé en ce que le fluide hydraulique (10) est recyclable.

4. Procédé suivant l'une quelconque des revendications 1 et 2, caractérisé en ce que le fluide hydraulique (10) est choisi parmi l'ensemble comprenant les milieux liquides notamment l'eau, les solutions salines, les huiles, les polyalkylèneglycols, les silicones et leurs mélanges.

5. Procédé suivant l'une quelconque des revendications 1 et 2, caractérisé en ce que le volume total de fluide hydraulique à introduire est sensiblement égal ou légèrement supérieur au volume de préparation médicamenteuse à distribuer.

6. Dispositif pour l'administration en continu d'une préparation médicamenteuse liquide, notamment par perfusion, par implantation ou par voie percutanée, ledit dispositif, qui comporte un système hydraulique et un réservoir et dans lequel le fluide hydraulique mis en circulation par la rétractation d'une membrane élastique préalablement dilatée assure, par l'intermédiaire d'un piston, la distribution en continu de ladite préparation contenue dans ledit réservoir vers son site d'administration, étant caractérisé en ce qu'il comprend dans ledit système hydraulique un accumulateur hydraulique sollicité par une membrane élastique (8) rétractable, un conduit capillaire (4) alimenté en fluide hydraulique par ledit accumulateur hydraulique, et un piston (12) disposé en aval dudit conduit capillaire et sollicité par le fluide hydraulique provenant dudit conduit capillaire, ledit piston étant destiné au transfert en continu de la préparation médicamenteuse contenue dans ledit réservoir (13) vers ledit site d'administration (15), ledit réservoir (13) étant logé dans un évidement longitudinal (110) débouchant à l'extérieur et creusé dans un corps cylindrique (1), ladite membrane élastique (8) étant montée de façon étanche sur au moins une portion de la surface extérieure (101) dudit corps cylindrique (1), ledit accumulateur hydraulique comprenant un fluide hydraulique (10) logeable dans l'espace (10a) situé entre (a) ladite membrane élastique dilatée par ledit fluide et (b) ladite portion de surface extérieure (101) dudit corps cylindrique (1) recouverte par ladite membrane élastique.

7. Dispositif suivant la revendication 6, caractérisé en ce qu'il comprend :

— un corps cylindrique (1) creusé d'un évidement longitudinal (110) débouchant à l'extérieur et dont le fond (210) présente une canalisation d'amenée (16) de fluide hydraulique,

— une membrane élastique (8) montée de façon étanche sur au moins une portion de la surface extérieure (101) dudit corps cylindrique (1),

— un accumulateur hydraulique comprenant un fluide hydraulique (10) logeable dans l'espace (10a) situé entre (a) ladite membrane élastique dilatée par ledit fluide et (b) ladite portion de surface extérieure (101) dudit corps cylindrique (1) recouverte par ladite membrane élastique,

— un conduit capillaire (4) destiné à être alimenté en fluide hydraulique par ledit accumulateur hydraulique, d'une part, et à alimenter ladite canalisation d'amenée disposée en aval dudit capillaire au fond de l'évidement dudit corps cylindrique, d'autre part,

— au moins un piston (12) situé dans ledit évidement (110) du corps cylindrique et susceptible de se déplacer axialement à l'intérieur dudit évidement, ledit piston comprenant une première face distale (112) directement sollicitée par le fluide hydraulique provenant de ladite canalisation d'amenée (16) de fluide et une seconde face distale (112) intervenant pour la distribution en continu de ladite préparation médicamenteuse vers son site d'administration (15),

— un réservoir tubulaire (13) contenant la préparation médicamenteuse liquide à administrer,

ledit réservoir et ledit système hydraulique étant agencés de telle façon que ledit réservoir tubulaire (13) soit solidaire du corps cylindrique (1) et associé audit piston (12).

8. Dispositif suivant la revendication 7, caractérisé en ce que le piston (12) se trouvant à l'intérieur de l'évidement (110) du corps cylindrique (1) est agencé de façon à être susceptible de coulisser à l'intérieur du réservoir tubulaire (13) contenant la préparation médicamenteuse (21).

9. Dispositif suivant la revendication 7, caractérisé en ce que le piston (12) se trouvant à l'intérieur de l'évidement (110) du corps cylindrique (1) est agencé de façon à solliciter par la pression du fluide hydraulique sur la paroi (112) un système de poussée (121, 221) agissant sur le contenu du réservoir (13), ledit système de poussée étant solidaire dudit réservoir (13), susceptible de coulisser à l'intérieur dudit réservoir (13) et logé au moins partiellement à l'intérieur dudit évidement (110).

10. Dispositif suivant la revendication 7, caractérisé en ce que la canalisation d'amenée (16) de fluide hydraulique du corps cylindrique (1) comporte sur au moins une partie de sa longueur une aiguille creuse (2) se prolongeant du côté de sa pointe effilée à l'intérieur de l'évidement (110) dudit corps cylindrique, ladite aiguille creuse (2) étant associée de façon étanche à ladite canalisation (16) et intervenant pour l'alimentation en fluide hydraulique de la paroi (112) du piston (12), d'une part, et pour la fixation du réservoir tubulaire (13) solidairement audit corps cylindrique (1), d'autre part.

11. Dispositif suivant la revendication 10, caractérisé en ce que la fixation du réservoir tubulaire (13) au corps cylindrique (1) au moyen de l'aiguille creuse (2) est réalisée grâce à un bouchon (11) obturant ledit réservoir (13) du côté opposé à celui de l'administration, ledit bouchon étant traversé de façon étanche par ladite aiguille, logé au moins sur une partie de son épaisseur dans l'intérieur dudit réservoir (13) et disposé en amont du piston (12) lequel est susceptible de coulisser de façon étanche à l'intérieur dudit réservoir (13), la pointe effilée de ladite aiguille (2) émergeant dudit bouchon au voisinage de la paroi (112) dudit piston.

12. Dispositif suivant la revendication 9, caractérisé en ce que le réservoir tubulaire (13) est monté dans une seringue, la préparation médicamenteuse (21) contenue dans ledit réservoir étant sollicité par la pression du fluide hydraulique exercée sur la paroi amont (112) du piston (12) et transmise par l'intermédiaire d'un mandrin (121) mû par la paroi amont (112a) dudit piston (12).

13. Dispositif suivant la revendication 12, caractérisé en ce que ledit mandrin (121) sollicite un piston (221) susceptible de coulisser à l'intérieur du réservoir (13), ledit réservoir étant solidaire dudit corps cylindrique (1) par l'intermédiaire d'un système du fixation (60, 62, 63) lié à l'ouverture extérieure de l'évidement (110) dudit corps cylindrique (1).

14. Dispositif suivant la revendication 7, caractérisé en ce que le conduit capillaire (4) a un diamètre régulier et est réalisé dans une pièce (44) logeable de façon étanche dans un évidement approprié du corps cylindrique (1) entre l'accumulateur hydraulique et la canalisation d'amenée (16).

15. Dispositif suivant la revendication 7, caractérisé en ce qu'il comprend outre l'évidement (110) au moins un autre évidement ouvert vers l'extérieur et opposé audit évidement (110) pour le logement d'un système de chargement (37) de l'accumulateur hydraulique en fluide hydraulique avant la mise en service.

16. Dispositif suivant l'une quelconque des revendications 6 et 7, caractérisé en ce que la membrane élastique (8) se présente sous la forme d'un manchon ou capuchon disposé autour du corps cylindrique (1), le diamètre dudit manchon ou capuchon à l'état non étiré étant inférieur à celui de la surface (101) dudit corps (1), le rapport :

$$R = \frac{\text{pression en charge} - \text{pression à vide}}{\text{Pression à vide}}$$

de ladite membrane (8) disposée autour dudit corps (1) étant compris entre 0,05 et 0,10 et avantageusement entre 0,06 et 0,09.

**Patentansprüche**

1. Verfahren zur Zuteilung eines flüssigen Medikaments in Richtung auf die Verabreichungsstelle, wobei die Verabreichung besonders durch Infusion, Implantation oder auf perkutanem Wege erfolgt, unter festgelegtem Durchfluß während einer relativ erheblichen Zeit und unter Ausnutzung der Retraktion einer elastischen Membran, dadurch **gekennzeichnet,** daß man das flüssige Medikament in einen röhrenförmigen Behälter (13) einbringt, worin es der Wirkung wenigstens einer unter dem Druck einer hydraulischen Flüssigkeit (10) verschiebbaren Wandung (112, 112a) unterworfen wird, deren Zufuhr über eine kapillare Leitung (4) gesteuert wird, in die die Einspeisung der hydraulischen Flüssigkeit unter Druck von einem Hydraulikspeicher erfolgt, der durch eine zusammenziehbare, vorher ausgeweitete elastische Membran belastet ist, wobei der Behälter (13) in einer longitudinalen, nach außen ragenden, in einem zylindrischen Körper (1) vertieften Aussparung (110) angeordnet ist, die elastische Membran (8) in abgedichtetem Zustand zumindest über einem Teil der äußeren Oberfläche (101) des zylindrischen Körpers (1) angebracht ist und der Hydraulikspeicher (10) hydraulische Flüssigkeit in dem Raum (10a) enthält, der gebildet ist zwischen (a) der elastischen, mit der Flüssigkeit ausgeweiteten Membran und (b) dem Teil der äußeren Oberfläche (101) des zylindrischen Körpers (1), der von der elastischen Membran bedeckt ist.

2. Verfahren gemäß Anspruch 1, dadurch **gekennzeichnet,** daß der Durchfluß des zu seinem Verabreichungsort zuzuteilenden Medikaments durch der Durchmesser der Kapillare (4) und die Viskosität der hydraulischen Flüssigkeit eingestellt wird.

3. Verfahren gemäß irgend einem der Ansprüche 1 und 2, dadurch **gekennzeichnet,** daß die hydraulische Flüssigkeit (10) rezyklisierbar ist.

4. Verfahren gemäß irgend einem der Ansprüche 1 und 2, dadurch **gekennzeichnet,** daß die hydraulische Flüssigkeit aus der von flüssigen Medien gebildeten Gruppe, besonders Wasser, Salzlösungen, Ölen, Polyalkylenglykolen, Siliconen und deren Mischungen ausgewählt ist.

5. Verfahren gemäß irgendeinem der Ansprüche 1 und 2, dadurch **gekennzeichnet,** daß das Gesamtvolumen der einzuführenden hydraulischen Flüssigkeit praktisch gleich oder etwa größer als das Volumen des zuzuteilenden Medikaments ist.

6. Vorrichtung zur fortlaufenden Verabreichung eines flüssigen Medikaments, insbesondere durch Infusion, Implantation oder perkutanes Vorgehen, die ein hydraulisches System und einen Behälter aufweist und in der die hydraulische Flüssigkeit, die durch das Zusammenziehen einer zunächst hinreichend ausgedehnten elastischen Membran bewegt wird, unter Vermittlung eines Kolbens die fortlaufende Zuteilung des Medikaments im Behälter in Richtung auf die Verabreichungsstelle sicherstellt, dadurch **gekennzeichnet,** daß sie in dem hydraulischen System einen durch eine elastische zusammenziehbare Membran (8) belasteten Hydraulikspeicher, eine kapillare Leitung (4), die vom Hyraulikspeicher mit hydraulischer Flüssigkeit gespeist wird, sowie einen Kolben (12) aufweist, der stromabwärtig der kapillaren Leitung angeordnet ist und durch die aus der kapillaren Leitung kommende hydraulische Flüssigkeit belastet wird, wobei der Kolben den kontinuierlichen Transfer des in dem Behälter (13) enthaltenen Medikaments in Richtung auf die Verabreichungsstelle (15) bewirkt, daß der Behälter (13) in einer longitudinalen, nach außen ragenden und in einem zylindrischen Körper (1) vertieften Aussparung (110) angeordnet ist, daß die elastische Membran (8) in abgedichtetem Zustand zumindest über einem Teil der äußeren Oberfläche des zylindrischen Körpers (1) angebracht ist und der Hydraulikspeicher (10) die hydraulische Flüssigkeit in dem Raum (10a) enthält, der zwischen (a) der elastischen, mit der Flüssigkeit ausgeweiteten Membran und (b) dem Teil der äußeren Oberfläche (101) des zylindrischen Körpers (1) liegt, der von der elastischen Membran bedeckt ist.

7. Vorrichtung gemäß Anspruch 6, dadurch **gekennzeichnet,** daß sie umfaßt :
— einen zylindrischen Körper (1), mit einer nach außen ragenden longitudinalen Aussparung (110) und einer Zuführleitung (16) für die hydraulische Flüssigkeit an deren Boden (210),
— eine elastische Membran (8), die in abgedichtetem Zustand über mindestens einem Teil der äußeren Oberfläche (101) des zylindrischen Körpers (1) angebracht ist,

— einen die hydraulische Flüssigkeit enthaltenden Hydraulikspeicher (10), angeordnet in dem Raum (10a), der gebildet ist zwischen (a) der elastischen, mit der Flüssigkeit ausgeweiteten Membran und (b) dem Teil der äußeren Oberfläche des zylindrischen Körpers, der von der elastischen Membran bedeckt ist,

— eine kapillare Leitung (4), bestimmt für die Einspeisung der hydraulischen Flüssigkeit aus dem Hydraulikspeicher einerseits und in die Zuführleitung am Boden der Aussparung des zylindrischen Körpers anderseits,

— wenigstens einen Kolben (12), gelegen in der Aussparung (110) des zylindrischen Körpers und geeignet für axiale Verschiebung im Inneren der Aussparung, von dem eine erste fernere Seite (112) direkt mit der hydraulischen Flüssigkeit aus der Zuführleitung (16) in Berührung steht und eine zweite Seite (112) die kontinuierliche Zuteilung des Medikaments in Richtung auf die Verabreichungsstelle (15) bewirkt,

— einen röhrenförmigen Behälter (13) zur Aufnahme des zu verabreichenden flüssigen Medikaments, wobei der Behälter und das hydraulische System derart angeordnet sind, daß der röhrenförmige Behälter (13) an dem zylindrischen Körper 1 dicht anliegt und mit dem Kolben (12) in Verbindung steht.

8. Vorrichtung gemäß Anspruch 7, dadurch **gekennzeichnet,** daß der im Innern der Aussparung (110) des zylindrischen Körpers (1) befindliche Kolben (12) derart angeordnet ist, daß er sich im Inneren des röhrenförmigen Behälters (13) mit dem Medikament (21) verschieben läßt.

9. Vorrichtung gemäß Anspruch 7, dadurch **gekennzeichnet,** daß der im Inneren der Aussparung (110) des zylindrischen Körpers (1) befindliche Kolben derart angeordnet ist, daß er durch den Druck der hydraulischen Flüssigkeit auf seine Wandung (112) ein Stoßsystem (121, 221) belastet, das auf den Inhalt des Behälters (13) wirkt, wobei das Stoßsystem solidarisch mit dem Behälter (13) ist und im Inneren des Behälters (13) verschoben werden kann und mindestens teilweise im Inneren der Aussparung (110) angeordnet ist.

10. Vorrichtung gemäß Anspruch 7, dadurch **gekennzeichnet,** daß die Zuführleitung (16) für die hydraulische Flüssigkeit des zylindrischen Körpers (1) auf mindestens einem Teil seiner Länge eine Hohlnadel (2) trägt, die von der Seite seiner abgeschrägten Spitze zum Inneren der Aussparung (110) des zylindrischen Körpers hinausragt und wobei die Hohlnadel (2) dicht mit der Leitung (16) verbunden ist und für die Speisung der hydraulischen Flüssigkeit zu der Wand (112) des Kolbens (12) einerseits und die solidarische Fixierung des röhrenförmigen Behälters (13) im zylindrischen Körper andererseits dient.

11. Vorrichtung gemäß Anspruch 10, dadurch **gekennzeichnet,** daß die Fixierung des röhrenförmigen Behälters (13) im zylindrischen Körper (1) mittels der Hohlnadel (2) verwirklicht wird durch einen Stöpsel (11), der den Behälter (13) auf der der Verabreichungsstelle gegenüberliegenden Seite verschließt, von der Nadel in abgedichteter Weise durchquert wird und zu mindestens einem Teil seiner Dicke im Behälter (13) angeordnet ist sowie stromaufwärts vom Kolben (12) liegt, der sich in abgedichteter Weise im Inneren des Behälters (13) verschieben läßt, und daß die abgeschrägte Spitze der Nadel (2) vom Stöpsel bis in die Nähe der Wandung (112) hervorragt.

12. Vorrichtung gemäß Anspruch 9, dadurch **gekennzeichnet,** daß der röhrenförmige Behälter (13) in einer Spritze angebracht ist, das Medikament (21) in dem Behälter durch den Druck belastet wird, der durch die hydraulische Flüssigkeit auf die stromaufwärtige Wandung (112) des Kolbens (12) wirkt und mittels einer Stange (121) in Verbindung mit der stromaufwärtigen Wandung (112a) des Kolbens (12) übertragen wird.

13. Vorrichtung gemäß Anspruch 12, dadurch **gekennzeichnet,** daß die Stange (121) einen Kolben (221) belastet, der sich im Inneren des Behälters (13) verschieben läßt, wobei der Behälter mit dem zylindrischen Körper (1) verbunden ist unter Zwischenschaltung eines Befestigungssystems (60, 62, 63), das an der äußeren Öffnung der Aussparung (110) des zylindrischen Körpers befestigt ist.

14. Vorrichtung gemäß Anspruch 7, dadurch **gekennzeichnet,** daß die kapillare Leitung (4) einen regelmäßigen Durchmesser hat und in einem Teil (44) realisiert ist, der in abgedichtetem Zustand in einer geeigneten Aussparung des zylindrischen Körpers (1) zwischen dem Hydraulikspeicher und der Zuführleitung (16) angeordnet ist.

15. Vorrichtung gemäß Anspruch 7, dadurch **gekennzeichnet,** daß sie noch außer der Aussparung (110) mindestens noch eine weitere nach außen offene und der Aussparung (110) gegenüberliegende Aussparung aufweist, die für die Anbringung eines Füllsystems (37) für den Hydraulikspeicher mit der hydraulischen Flüssigkeit vor der Inbetriebnahme dient.

16. Vorrichtung nach irgend einem der Ansprüche 6 und 7, dadurch **gekennzeichnet,** daß die elastische Membran (8) in der Form einer um den zylindrischen Körper (1) angeordneten Manschette oder Kappe vorliegt, wobei der Durchmesser der Manschette oder Kappe im nicht ausgedehnten Zustand kleiner als der der Oberfläche (101) des Körpers (1) ist und für die um den Körper (1) gelegte Membran (8) ein Verhältnis entsprechend

$$R = \frac{Druck\ unter\ Belastung\ -\ Druck\ bei\ Leere}{Druck\ bei\ Leere}$$

zwischen 0,05 und 0,10, vorzugsweise zwischen 0,06 und 0,09 besteht.

## Claims

1. A method for distributing towards its administration site a liquid medicinal preparation, said administration being able to be performed by perfusion, by implantation or percutaneously, at a determined rate for a relatively long period of time, the said method of distribution, which utilizes the retraction of an elastic membrane, comprising placing the said medicinal preparation in a tubular reservoir (13), where it is subjected to the action of at least one moving wall (112, 112a) driven by the pressure of a hydraulic fluid (10) whose admission is controlled by a capillary duct (4) supplied with hydraulic fluid (10) under pressure by a hydraulic accumulator driven by a retractable elastic membrane (8) dilated beforehand, the said reservoir (13) being housed in a longitudinal recess (110) leading to the outside and hollowed out of a cylindrical body (1), the said elastic membrane (8) being fixed in a leaktight manner to at least a portion of the outer surface (101) of the said cylindrical body (1), the said hydraulic accumulator comprising a hydraulic fluid (10) which can be accommodated in the space (10a) situated between (a) the said elastic membrane dilated by the said fluid, and (b) the said portion of outer surface (101) of the said cylindrical body (1) which is covered by the said elastic membrane.

2. The method according to claim 1, wherein the flow of the medicinal preparation to be distributed towards its administration site is regulated by the diameter of the capillary (4) and the viscosity of the hydraulic fluid.

3. The method according to claim 1 or claim 2, wherein the hydraulic fluid (10) can be recycled.

4. The method according to claim 1 or claim 2, wherein the hydraulic fluid (10) is selected from the group consisting of liquid media, especially water, saline solutions, oils, polyalkylene glycols, silicones and mixtures thereof.

5. The method according to claim 1 or claim 2, wherein the total volume of hydraulic fluid to be introduced is substantially equal to or slightly greater than the volume of medicinal preparation to be distributed.

6. A device for the continuous administration of a liquid medicinal preparation, especially by perfusion, by implantation or percutaneously, the said device — which has a hydraulic system and a reservoir and in which the hydraulic fluid circulated by the retraction of an elastic membrane dilated beforehand ensures, via a piston, continuous distribution of the said preparation, contained in the said reservoir, towards its administration site — comprising, in the said hydraulic system, a hydraulic accumulator driven by a retractable elastic membrane (8), a capillary duct (4) supplied with hydraulic fluid by the said hydraulic accumulator, and a piston (12) located downstream of the said capillary duct and driven by the hydraulic fluid coming from the said capillary duct, the said piston being intended for continuous transfer of the medicinal preparation, contained in the said reservoir (13), towards the said administration site (15), the said reservoir (13) being housed in a longitudinal recess (110) leading to the outside and hollowed out of a cylindrical body (1), the said elastic membrane (8) being fixed in a leaktight manner to at least a portion of the outer surface (101) of the said cylindrical body (1), the said hydraulic accumulator comprising a hydraulic fluid (10) which can be accommodated in the space (10a) situated between (a) the said elastic membrane dilated by the said fluid, and (b) the said portion of outer surface (101) of the said cylindrical body (1) which is covered by the said elastic membrane.

7. The device according to claim 6, which comprises :
— a cylindrical body (1) out of which a longitudinal recess (110) has been hollowed, the said recess leading to the outside and its bottom (210) having a channel (16) for the introduction of hydraulic fluid,
— an elastic membrane (8) fixed in a leaktight manner to at least a portion of the outer surface (101) of the said cylindrical body (1),
— a hydraulic accumulator comprising a hydraulic fluid (10) which can be accommodated in the space (10a) situated between (a) the said elastic membrane dilated by the said fluid, and (b) the said portion of outer surface (101) of the said cylindrical body (1) which is covered by the said elastic membrane,
— a capillary duct (4) intended on the one hand to be supplied with hydraulic fluid by the said hydraulic accumulator, and on the other hand to supply the said introduction channel located downstream of the said capillary at the bottom of the recess in the said cylindrical body,
— at least one piston (12) situated in the said recess (110) in the cylindrical body and capable of moving axially inside the said recess, the said piston comprising a first distal face (112) driven directly by the hydraulic fluid coming from the said fluid introduction channel (16), and a second distal face (112) used for continuous distribution of the said medicinal preparation towards its administration site (15), and

— a tubular reservoir (13) containing the liquid medicinal preparation to be administered,

the said reservoir and the said hydraulic system being designed in such a way that the said tubular reservoir (13) is integral with the cylindrical body (1) and associated with the said piston (12).

8. The device according to claim 7, wherein the piston (12) located inside the recess (110) in the cylindrical body (1) is designed so as to be capable of sliding inside the tubular reservoir (13) containing the medicinal preparation (21).

9. The device according to claim 7, wherein the piston (12) located inside the recess (110) in the cylindrical body (1) is designed so as to drive, by means of the pressure of the hydraulic fluid on its wall (112), a thrust system (121, 221) acting on the contents of the reservoir (13), the said thrust system being integral with the said reservoir (13), capable of sliding inside the said reservoir (13) and housed at least partially inside the said recess (110).

10. The device according to claim 7, wherein the channel (16) for the introduction of hydraulic fluid, in the cylindrical body (1), has, over at least part of its length, a hollow needle (2) which extends, at its tapered point, inside the recess (110) in the said cylindrical body, the said hollow needle (2) being associated in a leaktight manner with the said channel (16) and serving on the one hand to supply the wall (112) of the piston (12) with hydraulic fluid, and on the other hand to fix the tubular reservoir (13) firmly to the said cylindrical body (1).

11. The device according to claim 10, wherein the fixing of the tubular reservoir (13) to the cylindrical body (1) by means of the hollow needle (2) is effected by virtue of a stopper (11) blocking the said reservoir (13) on the opposite side to that of administration, the said stopper, through which the said needle passes in a leaktight manner, being housed, over at least part of its thickness, in the interior of the said reservoir (13) and located upstream of the piston (12), which is capable of sliding in a leaktight manner inside the said reservoir (13), the tapered point of the said needle (2) coming out of the said stopper in the vicinity of the wall (112) of the said piston.

12. The device according to claim 9, wherein the tubular reservoir (13) is fitted into a syringe, the medicinal preparation (21) contained in the said reservoir being driven by the pressure of the hydraulic fluid exerted on the upstream wall (112) of the piston (12) and transmitted via a plunger (121) actuated by the upstream wall (112a) of the said piston (12).

13. The device according to claim 12, wherein the said plunger (121) drives a piston (221) capable of sliding inside the reservoir (13), the said reservoir being integral with the said cylindrical body (1) via a fixing system (60, 62, 63) joined to the outside opening in the recess (110) in the said cylindrical body (1).

14. The device according to claim 7, wherein the capillary duct (4) has a uniform diameter and is made in a part (44) which can be accommodated in a leaktight manner in an appropriate recess in the cylindrical body (1), between the hydraulic accumulator and the introduction channel (16).

15. The device according to claim 7, which comprises, in addition to the recess (110), at least one other recess, open to the outside and opposite the said recess (110), for housing a system (37) for charging the hydraulic accumulator with hydraulic fluid before the device is brought into service.

16. The device according to claim 6 or claim 7, wherein the elastic membrane (8) is in the form of a sleeve or cap placed around the cylindrical body (1), the diameter of the said sleeve or cap in the unstretched state being less than that of the surface (101) of the said body (1) and the ratio :

$$R = \frac{\text{pressure when charged} - \text{pressure when empty}}{\text{pressure when empty}}$$

of the said membrane (8) placed around the said body (1) being between 0.05 and 0.10 and advantageously between 0.06 and 0.09.

14

FIG_1

FIG_2

FIG_3

FIG_4

## FIG_6

60    62    61   Y    8    X    112   210   44   4   3

122a   15 ←   13

5
7
2
17
9'

60a   221   222   63   9   121   122   112a   12   73   16   71   74  
70   72

EP 0 329 784 B1

## FIG_5

13
21
23
12
22
11

## FIG_7

15
150
152   153
151

## FIG_8

402
400   404
44
401
403